(19) Europäisches Patentamt
European Patent Office
Office européen des brevets

(11) **EP 3 873 335 B1**

(12) **EUROPEAN PATENT SPECIFICATION**

(45) Date of publication and mention
of the grant of the patent:
**31.12.2025 Bulletin 2026/01**

(21) Application number: **19880039.3**

(22) Date of filing: **02.11.2019**

(51) International Patent Classification (IPC):
**A61B 5/00** *(2006.01)*      **A61N 1/36** *(2006.01)*
**A61N 1/372** *(2006.01)*

(52) Cooperative Patent Classification (CPC):
**A61N 1/36135; A61B 5/4836;** A61B 5/407;
A61N 1/36062; A61N 1/36139; A61N 1/36146;
A61N 1/37241; A61N 1/37247

(86) International application number:
**PCT/AU2019/051210**

(87) International publication number:
**WO 2020/087135 (07.05.2020 Gazette 2020/19)**

(54) **CHARACTERISATION OF NEUROSTIMULATION THERAPEUTIC EFFICACY**

CHARAKTERISIERUNG VON THERAPEUTISCHER WIRKSAMKEIT DER NEUROSTIMULATION

CARACTÉRISATION DE L'EFFICACITÉ THÉRAPEUTIQUE D'UNE NEUROSTIMULATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.11.2018 US 201862754861 P**

(43) Date of publication of application:
**08.09.2021 Bulletin 2021/36**

(73) Proprietor: **Saluda Medical Pty Ltd
Artarmon, New South Wales 2064 (AU)**

(72) Inventors:
• **PARKER, John Louis
Artarmon, NSW 2064 (AU)**
• **SINGLE, Peter Scott Vallack
Artarmon, NSW 2064 (AU)**

(74) Representative: **Abel & Imray LLP
Westpoint Building
James Street West
Bath BA1 2DA (GB)**

(56) References cited:
WO-A1-2012/155188      WO-A1-2012/162349
WO-A1-2014/071446      WO-A1-2016/077882
WO-A1-2017/173493      WO-A1-2017/219096
WO-A1-2018/119220      WO-A1-2018/160992
WO-A2-2005/122887      US-A1- 2010 057 159
US-A1- 2016 082 265      US-A1- 2016 101 289

## Description

Technical Field

The present invention relates to therapeutic efficacy of neurostimulation, and in particular to methods and devices for assessing therapeutic efficacy, and for revising therapy and device operation to improve therapeutic efficacy.

Background of the Invention

There are a range of situations in which it is desirable to apply neural stimuli in order to give rise to a compound action potential (CAP). For example, neuromodulation is used to treat a variety of disorders including chronic pain, Parkinson's disease, and migraine. A neuromodulation system applies an electrical pulse to tissue in order to generate a therapeutic effect. When used to relieve chronic pain, the electrical pulse is typically applied to the dorsal column (DC) of the spinal cord, referred to as spinal cord stimulation (SCS). Neuromodulation systems typically comprise an implanted electrical pulse generator, and a power source such as a battery that may be rechargeable by transcutaneous inductive transfer. An electrode array is connected to the pulse generator, and is positioned in the dorsal epidural space above the dorsal column. An electrical pulse applied to the dorsal column by an electrode causes the depolarisation of neurons, and generation of propagating action potentials.

While the clinical effect of spinal cord stimulation (SCS) is well established, the precise mechanisms involved are poorly understood. Consequently, despite the invasive nature of implantation surgery, and the expense of such devices and procedures, there remains a trial-and-error aspect to the selection of patients who might benefit from such implants. Prospective implant recipients typically undergo an initial trial period in which only the electrode leads are implanted and the control unit is not implanted, with control being effected during the trial by way of transcutaneous lead connections. Such trials thus carry the attendant risks of infection, and the costs of managing such a configuration, as are known to be associated with transcutaneous lead configurations.

At the end of the trial period some patients will be deemed to receive inadequate therapeutic efficacy from the neurostimulation. The trial is then deemed a failure and the permanent implantation does not proceed, so that the cost and risks of the trial are thus unrewarded. This occurs for a significant proportion of SCS trials, in excess of 10%.

Moreover, for the cohort of patients for whom an SCS trial is deemed a success, and for whom permanent implantation does proceed, there nevertheless remains a significant attrition rate. A large proportion of such patients undergo subsequent explantation, such as around a quarter of SCS implant recipients. Among such explants, the dominant reason for the explantation is typically a lack of therapeutic efficacy, being the case in up to 40-45% of explants.

Despite the significant proportion of failed SCS trials, and the significant proportion of failed SCS implants due to a lack of therapeutic efficacy, there exists no reliable approach for predicting whether a given candidate will respond to the SCS therapy and achieve a sufficient therapeutic effect. To date, efforts to identify whether a candidate will be an SCS responder or an SCS non-responder typically have focused on the patient's demographics and diagnosis. Moreover, assessment of the efficacy of SCS has been limited to subjective outcomes such as the Visual Analogue Scale (VAS). Despite many decades of implantation of neurostimulators, there has been little success in improving responder rates in that time. WO 2016/077882 discloses a method and device for detecting a neural response in neural measurements, so as to detect whether a locally evoked neural response is present in the neural measurement.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed before the priority date of each claim of this application.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

In this specification, a statement that an element may be "at least one of" a list of options is to be understood that the element may be any one of the listed options, or may be any combination of two or more of the listed options.

Summary of the Invention

The invention is defined by the independent claims. According to a first aspect the present invention provides a system for assessing therapeutic efficacy of electrical neurostimulation therapy, the system comprising:

a neuromodulation device configured to deliver the electrical neurostimulation therapy;
measurement circuitry configured to capture recordings of neural responses evoked by the electrical neurostimulation therapy; and

a processor configured to process the recordings of the neural responses evoked by the delivered electrical neurostimulation therapy, in order to obtain a plurality of measures of neural activation in response to a plurality of electrical stimuli delivered over time, the processor further configured to calculate from the plurality of measures of neural activation in response to the plurality of delivered electrical stimuli at least one statistical measure of neural activation by the plurality of delivered electrical stimuli, the processor further configured to produce from the at least one statistical measure of neural activation by the plurality of delivered electrical stimuli an indication of therapeutic efficacy, the processor further configured to output the indication of therapeutic efficacy.

According to a second example which is not covered by the subject matter of the claims, there is provided a method for assessing therapeutic efficacy of electrical neurostimulation therapy, the method comprising:

delivering electrical neurostimulation therapy;
capturing recordings of neural responses to the electrical neurostimulation therapy;
processing the recordings of the neural responses in order to obtain a plurality of measures of neural activation in response to a plurality of stimuli delivered over time,
calculating from the plurality of measures of neural activation at least one statistical measure of neural activation,
producing from the at least one statistical measure an indication of therapeutic efficacy; and
outputting the indication of therapeutic efficacy.

According to a third aspect the present invention provides a non-transitory computer readable medium for assessing therapeutic efficacy of electrical neurostimulation therapy, comprising instructions which, when executed by one or more processors, causes performance of the following:

delivering, by a stimulation electrode of a stimulator device, electrical neurostimulation therapy;
capturing, by a sense electrode of the stimulator device, recordings of neural responses evoked by the electrical neurostimulation therapy;
processing, by the one or more processors, the recordings of the neural responses evoked by the delivered electrical neurostimulation therapy, in order to obtain a plurality of measures of neural activation in response to a plurality of electrical stimuli delivered over time,
calculating, by the one or more processors, from the plurality of measures of neural activation in response to the plurality of delivered electrical stimuli at least one statistical measure of neural activation by the plurality of delivered electrical stimuli,
producing, by the one or more processors, from the at least one statistical measure of neural activation by the plurality of delivered electrical stimuli an indication of therapeutic efficacy; and
outputting, by the one or more processors, the indication of therapeutic efficacy.

In some embodiments of the invention, the measures of neural activation comprise ECAP amplitude measures, for example a measure of N1-P2 peak to peak amplitude. Such ECAP amplitude measures may be considered in the form of an ECAP amplitude histogram.

In some embodiments of the invention the at least one statistical measure calculated from the plurality of measures comprises one or more of: a mode of the plurality of measures, a mode/comfort+ ratio of the plurality of measures, a normalised interdecile range of the plurality of measures, a measure of distribution asymmetry such as a mean/median ratio of the plurality of measures, a measure of dispersion of the plurality of measures such as a coefficient of variation of the plurality of measures, a kurtosis of the plurality of measures, and/or a skew of the plurality of measures. Comfort+ herein refers to a clinician prescribed level of desirable neural activation.

Additionally or alternatively, some embodiments of the present invention may normalise the plurality of measures prior to determining the at least one statistical measure. Normalisation may comprise dividing each measure by a comfort+, mean, median or mode of the plurality of measures. Normalisation recognises that significant variation can arise in measures of absolute ECAP amplitude from one patient to the next, and even for a given patient such variations can depend on factors including a stimulus regime, a distance of the stimulus electrode(s) from the recording electrode(s), a distance of the recording electrode(s) from the nerve whether caused by short term postural changes or by longer term lead migration, and other such factors. The application of normalisation and/or the use of statistical measures which do not depend on absolute ECAP amplitude permits comparisons of therapeutic efficacy to be validly made between patients and/or for a given patient experiencing changing therapy parameters over time.

In one preferred embodiment the plurality of measures are measures of ECAP amplitude in volts at the patient's comfort + level, in a pre-determined posture, and the at least one statistical measure comprises a coefficient of variation of the plurality of measures. Such embodiments recognise that the width of the histogram is somewhat constant from patient to patient, and may in some cases be limited by hardware, and further recognise that the coefficient of variation in turn is inversely related to the ECAP amplitude. The present invention further recognises that a small dispersion of the plurality of measures, corresponding to a small coefficient of variation, is associated with improved therapeutic efficacy.

Preferably, two or more such statistical measures are derived from the plurality of measures of neural activation, and used to produce a combinatorial indication of therapeutic efficacy.

In further embodiments of the invention the at least one statistical measure may comprise any of the above noted measures, compensated for a distance-dependent transfer function of stimulation, and/or compensated for a distance dependent transfer function of measurement. Such distance dependent transfer function compensation may be implemented in the manner described in the present Applicant's International Patent Publication No. WO2017173493. Any measure of neural activation, such as a measure generated in accordance with the teachings of WO2017173493 or a measure generated by any other suitable means, may form the basis of the at least one statistical measure in accordance with some embodiments of the present invention.

In some embodiments, producing the indication of therapeutic efficacy from the at least one statistical measure may involve comparing the observed statistical measure to a predefined reference, such as a corresponding measure derived from a control population.

In some embodiments of the invention the method is performed during a neurostimulation device trial, and the indication of therapeutic efficacy is used as a binary indicator to indicate whether permanent implantation should proceed.

In some embodiments of the invention the method is performed by a permanent implant and the indication of therapeutic efficacy is used to refine implant operation over time to guide future therapy and/or to predict an end-of-usefulness of the implant as the indication of therapeutic efficacy deteriorates. In such embodiments the measurement circuitry is preferably within the implant. The measurement circuitry may operate in accordance with the teachings of the present Applicant's International Patent Publication No. WO2012155183. The processor for processing the recordings of the neural responses may be within the implant or may be part of an external device which receives the recordings from the implant.

In some embodiments of the invention the stimulus electrodes and measurement electrodes are a part of the same electrode array.

The present invention thus identifies a relationship between diagnostic markers of neural activation, on the one hand, and measuring or predicting response / non-response to neurostimulation such as SCS, on the other hand, and further provides for application of such a relationship in order to revise device configuration and/or otherwise optimise therapy.

The indication of therapeutic efficacy in some embodiments may be used to optimise therapy by guiding changes to selection of the stimulating electrode(s). The indication of therapeutic efficacy in some embodiments may be used to optimise therapy by guiding changes to selection of recording electrode(s). The indication of therapeutic efficacy in some embodiments may be used to optimise therapy by guiding changes to selection of a combination of recording and stimulating electrodes. The indication of therapeutic efficacy in some embodiments may be used to optimise therapy by guiding changes to selection of a stimulus intensity, selection of a stimulus current, or selection of stimulus pulse width(s). The indication of therapeutic efficacy in some embodiments may be used to optimise therapy by guiding changes to selection of measurement amplifier settings. The indication of therapeutic efficacy in some embodiments may be used to optimise therapy by guiding changes to selection of a target level of neural activation for example based on a pre-determined relationship relative to the statistical measure(s), a threshold level and a patient comfort level. The indication of therapeutic efficacy in some embodiments may be used to optimise therapy by guiding changes to selection of feedback loop implementation such as selection among feedback loop implementations set out in WO2017173493. The indication of therapeutic efficacy in some embodiments may be used to optimise therapy by guiding changes to selection of feedback loop parameters, such as feedback loop gain, feedback loop noise bandwidth, and feedback loop instant backoff threshold. Additionally or alternatively, the indication of therapeutic efficacy in some embodiments may be used to optimise therapy by identifying a patient as a responder or a non-responder, and/or may be used to guide a decision as to whether to continue or cease treatment.

Further embodiments of the invention may comprise an automated procedure for improving therapeutic efficacy, by iteratively revising the therapy to seek an improvement in the indication of therapeutic efficacy. Any suitable systematic method of revising patient settings that improved the indication of therapeutic efficacy may be selected. For example, a feedback loop gain may be optimised by an iterative process involving: (i) measuring a first indication of therapeutic efficacy with feedback loop gain set at a first value, (ii) adjusting feedback loop gain from a first value to a second value, (iii) measuring a second indication of therapeutic efficacy and (iv) if the second indication indicates higher therapeutic efficacy than the first indication, retaining the second value of loop gain for ongoing use. Such an iterative procedure may be repeated any number of times required to sufficiently explore the available range of options for the feedback loop gain and to find an optimal value. A corresponding iterative procedure may be performed in respect of any aspect of operation of the device.

The indication of therapeutic efficacy in some embodiments may be normalised to compensate for variations caused by postural changes, or physiological events such as coughs, heartbeat and breathing.

In some embodiments of the invention, the measurement circuitry is configured to record the recordings of the neural responses substantially continuously during device operation. For example, in some embodiments of the invention the implanted neuromodulation device is configured to record the recordings of the neural responses for a period of at least 8 hours of device operation. In some embodiments of the invention the implanted neuromodulation device is configured to record the recordings of the neural responses for a period of at least 2 days of device operation. In some embodiments of the invention the implanted neuromodulation device is configured to record the recordings of the neural responses for a period of at least 5 days of device operation. To this end, preferred embodiments of the invention provide for the implanted neuromodulation device to be configured to process each recording of a neural response in substantially real time in order to obtain a respective measure of neural activation, and further provide for the implanted neuromodulation device to store in memory only the measure of neural activation and not the entire recording. For example, the implanted neuromodulation device may store in memory a histogram of the plurality of measures of neural activation in the form of a plurality of bins, with a counter associated with a respective bin being incremented each time an additional measure of neural activation is obtained. Such embodiments permit such data to be obtained over a period of hours or days at a high rate, such as at 50 Hz or more, and to be stored in very compact manner by use of a histogram and to thereby avoid exceeding the limited memory constraints of an implantable device.

References herein to estimation, determination, comparison and the like are to be understood as referring to an automated process carried out on data by a processor operating to execute a predefined procedure suitable to effect the described estimation, determination and/or comparison step(s). The approaches presented herein may be implemented in hardware (e.g., using application specific integrated circuits (ASICS)), or in software (e.g., using instructions tangibly stored on computer-readable media for causing a data processing system to perform the steps described herein), or in a combination of hardware and software. The invention can also be embodied as computer-readable code on a computer-readable medium. The computer-readable medium can include any data storage device that can store data which can thereafter be read by a computer system. Examples of the computer readable medium include read-only memory ("ROM"), random-access memory ("RAM"), CD-ROMs, DVDs, magnetic tape, optical data storage device, flash storage devices, or any other suitable storage devices. The computer-readable medium can also be distributed over network coupled computer systems so that the computer readable code is stored and executed in a distributed fashion.

Brief Description of the Drawings

An example of the invention will now be described with reference to the accompanying drawings, in which:

Fig. 1 schematically illustrates an implanted spinal cord stimulator;
Fig. 2 is a block diagram of the implanted neurostimulator;
Fig. 3 is a schematic illustrating interaction of the implanted stimulator with a nerve;
Fig 4A and 4B are plots of a series of recordings of ECAP amplitude obtained in closed loop mode, and open loop mode, respectively;
Fig. 5 illustrates an alternative presentation of clinical data
Fig. 6 illustrates a histogram of neural activation data
Fig. 7 illustrates the respective VAS reduction observed in two groups of SCS trial participants.
Fig. 8 illustrates the average mode of ECAP amplitude for each SCS trial group.
Fig. 9 illustrates the average of the mode/comfort+ ratio for each SCS trial group.
Fig. 10 illustrates the average of the (90th-10th)/median ratio for each SCS trial group.
Fig. 11 illustrates the average of the mean/median ratio for each SCS trial group.
Fig. 12 illustrates an intraoperative procedure in accordance with a further embodiment of the invention
Fig. 13 illustrates an SCS trial procedure in accordance with another embodiment of the invention;
Fig. 14 illustrates an SCS implant procedure in accordance with yet another embodiment of the invention;
Fig. 15 is a dose response plot for both neurostimulation benefit and detriment;
Fig. 16 is a dose response plot also showing a first example of a broad histogram of neural measures and the resulting net benefit and net detriment;
Fig. 17 is a dose response plot also showing a second example of a broad histogram of neural measures and the resulting net benefit and net detriment;
Fig. 18 is a dose response plot also showing a third example of a broad histogram of neural measures and the resulting net benefit and net detriment;
Fig. 19 is a dose response plot also showing an example of a narrow histogram of neural measures and the resulting net benefit and net detriment; and
Fig. 20 is a plot of summed net benefit and detriment for open loop neurostimulation, for closed loop stimulation achieving a broad histogram, and for closed loop stimulation achieving a narrow histogram.

Description of the Preferred Embodiments

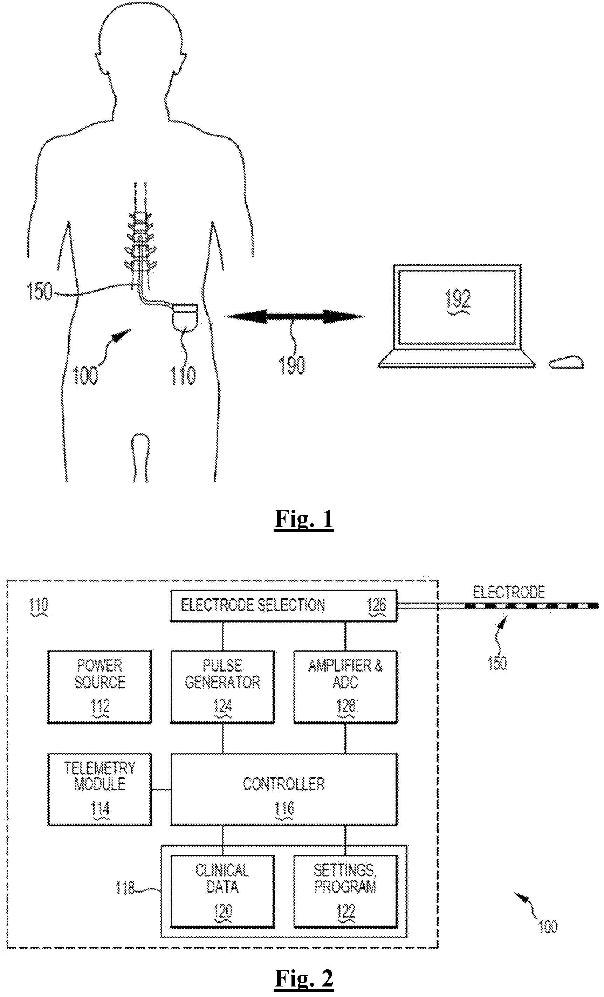

**Fig. 1** schematically illustrates an implanted spinal cord stimulator 100. Stimulator 100 comprises an electronics module 110 implanted at a suitable location in the patient's lower abdominal area or posterior superior gluteal region, and an electrode assembly 150 implanted within the epidural space and connected to the module 110 by a suitable lead. Numerous aspects of operation of implanted neural device 100 are reconfigurable by an external control device 192. Moreover, implanted neural device 100 serves a data gathering role, with gathered data being communicated to external device 192 via any suitable transcutaneous communications channel 190.

**Fig. 2** is a block diagram of the implanted neurostimulator 100. Module 110 contains a battery 112 and a telemetry module 114. In embodiments of the present invention, any suitable type of transcutaneous communication 190, such as infrared (IR), electromagnetic, capacitive and inductive transfer, may be used by telemetry module 114 to transfer power and/or data between an external device 192 and the electronics module 110. Module controller 116 has an associated memory 118 storing patient settings 120, control programs 122 and the like. Controller 116 controls a pulse generator 124 to generate stimuli in the form of current pulses in accordance with the patient settings and control programs 122. Electrode selection module 126 switches the generated pulses to the appropriate electrode(s) of electrode array 150, for delivery of the current pulse to the tissue surrounding the selected electrode(s). Measurement circuitry 128 is configured to capture measurements of neural responses sensed at sense electrode(s) of the electrode array as selected by electrode selection module 126.

It is to be noted that in alternative embodiments of the invention, the electronics module 110 may be outside the body, for example in the form of a trial SCS controller, or an intraoperative control device. In such alternative embodiments the power source 112 may be substituted with mains power, and telemetry module 114 may be simplified or substituted by other forms of data connection modules.

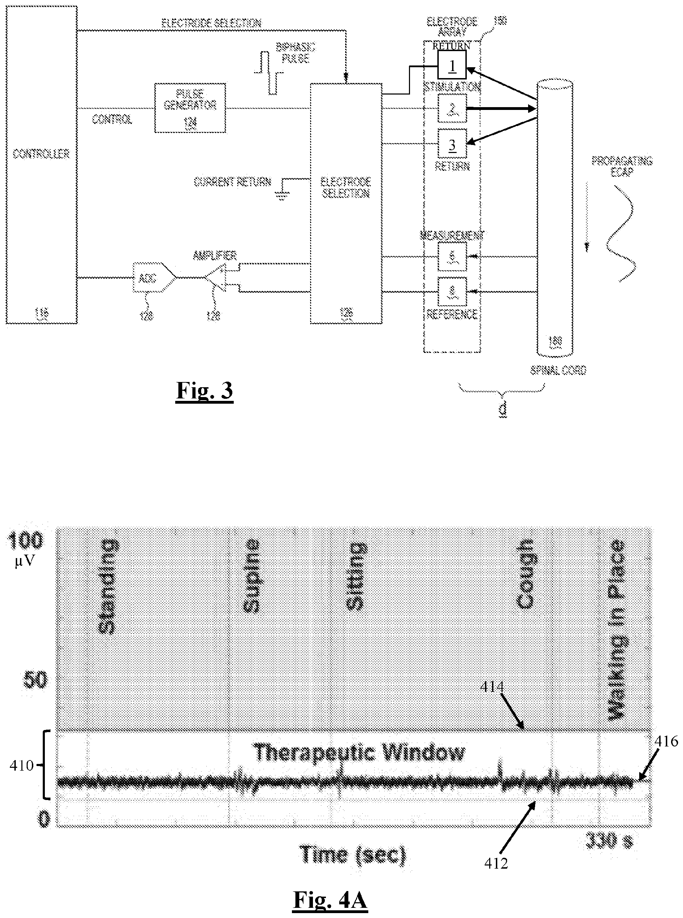

**Fig. 3** is a schematic illustrating interaction of the implanted stimulator 100 with a nerve 180, in this case the spinal cord however alternative embodiments may be positioned adjacent any desired neural tissue including a peripheral nerve, visceral nerve, parasympathetic nerve or a brain structure. Electrode selection module 126 selects a stimulation electrode 2 of electrode array 150 to deliver a biphasic tripolar electrical current pulse to surrounding tissue including nerve 180. Electrode selection module 126 also selects two return electrodes 1 and 3 of the array 150 for stimulus current recovery to maintain a zero net charge transfer. Alternative embodiments may employ alternative stimulus configuration(s), such as tripolar triphasic stimulation, for the reasons set out in the present Applicant's International Patent Publication No. WO 2017219096 A1.

Delivery of an appropriate stimulus to the nerve 180 evokes a neural response comprising a compound action potential which will propagate along the nerve 180 as illustrated, for therapeutic purposes which in the case of a spinal cord stimulator for chronic pain might be to create paraesthesia at a desired location. To this end the stimulus electrodes are used to deliver stimuli at any therapeutically suitable frequency, for example 30 Hz, although other frequencies may be used including as high as the kHz range, and/or stimuli may be delivered in a non-periodic manner such as in bursts, or sporadically, as appropriate for the patient. To fit the device, a clinician applies stimuli of various configurations which seek to produce a sensation that is experienced by the user as a paraesthesia. When a stimulus configuration is found which evokes paraesthesia, which is in a location and of a size which is congruent with the area of the user's body affected by pain, the clinician nominates that configuration for ongoing use.

The device 100 is further configured to sense the existence and intensity of compound action potentials (CAPs) propagating along nerve 180, whether such CAPs are evoked by the stimulus from electrodes 1-3, or otherwise evoked. To this end, any electrodes of the array 150 may be selected by the electrode selection module 126 to serve as measurement electrode 6 and measurement reference electrode 8. Signals sensed by the measurement electrodes 6 and 8 are passed to measurement circuitry 128, which for example may operate in accordance with the teachings of International Patent Application Publication No. WO2012155183 by the present applicant. The output of circuitry 128 is used by controller 116 in a feedback arrangement to control the application of subsequent stimuli, and the controller 116 also stores the recording of the neural response, or one or more parameters thereof such as ECAP amplitude, to the Clinical Data storage 120.

Stimulator 100 applies stimuli over a potentially long period such as days or weeks, and records neural responses, stimulation settings, paraesthesia target level, and other operational parameters, discussed further below. The stimulator 100 comprises a closed loop stimulator (CLS), in that the recorded neural responses are used in a feedback arrangement to control stimulation settings on a continuous or ongoing basis. To effect suitable SCS therapy stimulator 100 may deliver tens, hundreds or even thousands of stimuli per second, for many hours each day. The feedback loop may operate for most or all of this time, by obtaining neural response recordings following every stimulus, or at least obtaining such recordings sufficiently regularly that the feedback loop can respond in a timely manner, for example to respond to postural changes of the user. Each recording generates a feedback variable such as a measure of the amplitude of the evoked neural response, which in turn results in the feedback loop changing the stimulation parameters for a following stimulus if required. Stimulator 100 thus produces such data at a rate of tens or hundreds of Hz, or even kHz, and over the course of hours or days this process results in large amounts of clinical data. This is unlike past neuromodulation devices such as SCS devices which lack any ability to record any neural response.

When brought in range with a receiver, or when operating during a programming session under control of an external device of a clinician, stimulator 100 transmits data via telemetry module 114 to a clinical programming application, which compiles a clinical data log file which can be manipulated and optimised and presented by a clinical data viewer for field diagnosis by the clinician or field clinical engineer (FCE). The software application used to analyse the data generated by the stimulator 100 is installed on a Clinical Interface (CI) tablet computer, or on another computer 192 running any suitable operating system such as Microsoft Windows. The data can be grouped into two main sources: 1. Data collected in real-time during a programming session, and 2. Data downloaded from a stimulator after a period of non-clinical use by a patient.

The present invention recognises that the mechanisms of action (MoA) for SCS propose an inhibitory effect of spinal cord stimulation on neuropathic pain, with activated axons likely responsible for the paresthesia of conventional SCS. The present invention further recognises that the evoked compound action potential (ECAP) recordings obtained by the implant 100, or by a trial device with transcutaneous leads, can be assessed and serve to elucidate that SCS activates Aβ fibres in the dorsal column. This enables a patient's electrophysiological response to SCS stimulation, such as ECAP amplitude, to be correlated with pain relief. This in turn facilitates closed loop SCS, whereby ECAP amplitude can be maintained within a narrow therapeutically effective range, referred to herein as the therapeutic window (TW). **Fig 4A** is a plot of a series of recordings of ECAP amplitude obtained over approximately 6 minutes, obtained by device 100 when operating in closed loop mode. As can be seen, despite the patient undertaking a range of physical and physiological tasks including standing, lying supine, sitting, coughing and walking in place, the closed loop mode is highly effective at maintaining the ECAP amplitude within the therapeutic window 410. The therapeutic window 410 is defined herein as residing between a perception threshold 412 and a maximum level 414. The perception threshold 412 is the minimum ECAP amplitude which is subjectively perceived by the patient, which as can be seen in Fig 4A is a non-zero ECAP amplitude level. The maximum level 414 is the largest ECAP amplitude which the patient can comfortably tolerate. The closed loop mode feedback is configured to produce ECAPs at a target level 416 within the therapeutic range, the target level 416 being the ECAP level at which the patient reports comfortable paresthesia.

The therapeutic window 410 can also be defined as residing between an ECAP threshold 412 and a maximum level 414. The ECAP threshold 412 is the minimum current amplitude at which an ECAP can be detected.

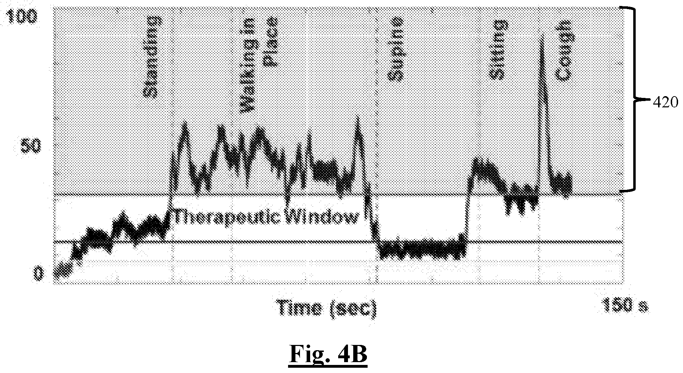

This is in contrast to conventional SCS which operates without feedback control, in what is referred to as an open loop mode. **Fig 4B** is a plot of a series of recordings of ECAP amplitude over an approximately 2 minutes period of time, obtained by device 100 when operating in open loop mode. In open loop mode, at any given setting of stimulus intensity, significant ECAP amplitude variation can result from normal physical and physiological activity, as shown in Fig. 4B. As can be seen, as the patient undertakes a range of physical and physiological tasks including standing, walking in place, lying supine, sitting and coughing, the open loop mode is ineffective at maintaining the ECAP amplitude within the therapeutic window 410. Open loop mode SCS thus often causes overstimulation, whereby the evoked ECAP amplitude exceeds the therapeutic window. The overstimulation range is indicated at 420. The present invention recognises that such overstimulation can result in repeated Aβ nociceptor activation which in turn may cause spinal cord sensitisation. And, spinal cord sensitisation reduces or negates the pain inhibition afforded by SCS, leading to a loss of therapeutic benefit.

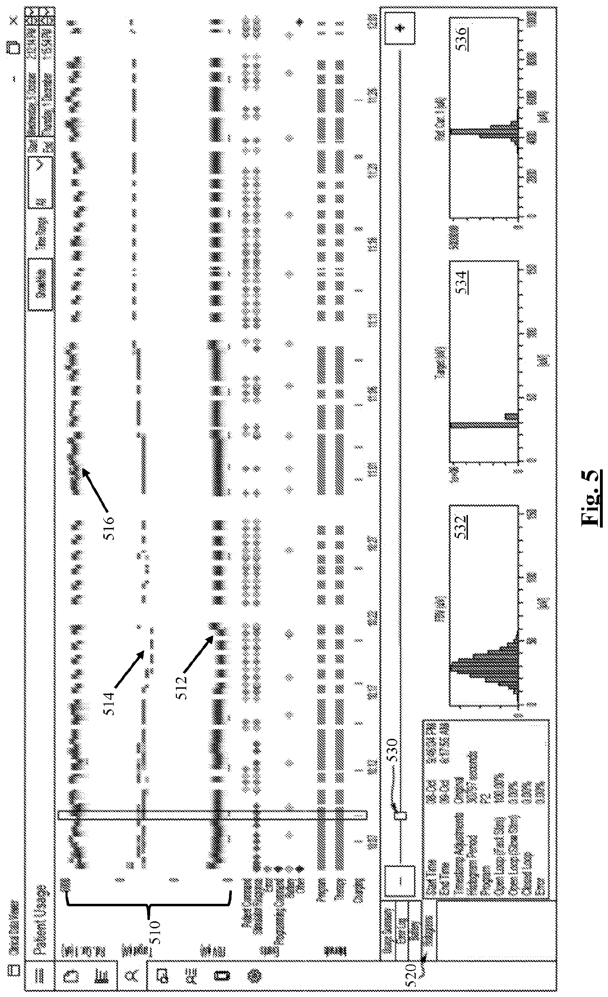

**Fig. 5** illustrates an alternative presentation of clinical data from device 100, for a non-clinical usage period of about two hours, effected by the Clinical Data Viewer software. Therapy Logs 510 are displayed, in the form of a heat-map. Each heat map is a representation of the proportion of stimuli (intensity) which had a particular magnitude (y-axis) at each time point (x-axis), as derived from a histogram of the data retrieved from the device 100 over a short period of time. For example, each such histogram may be compiled over a respective 2 minutes time period. The more intense the heat map, the greater the proportion of stimuli with that magnitude at that time. Three heat maps are displayed, Feedback Variable 512, Feedback Target 514, and Stimulus Current 516. The feedback variable for example could be observed ECAP amplitude as measured as being the peak to peak value from N1 to P2 of each measured ECAP. The Feedback Variable 512 heat map is thus an alternative display method for ECAP amplitude data of the type shown in Figs 4A and 4B, being conducive to the display of larger such data sets obtained over a longer period of time.

Within tab 520, slider bar 530 is a graphical user interface which allows the reviewer to select a temporal subset of the data 510. For that selected subset of data, the clinical data viewer extracts histogram data for the feedback variable as shown in plot 532, and extracts histogram data for the feedback target as shown in plot 534, and extracts histogram data for the stimulus current as shown in plot 536, for closer assessment. The histogram extracted from each short period of data is also presented as Therapy Logs 510, which show the individual histograms in heat map form over the entire 2 hours period, using a heat-map to efficiently represent the column heights of each histogram 532, 534, 536, for each segment of time.

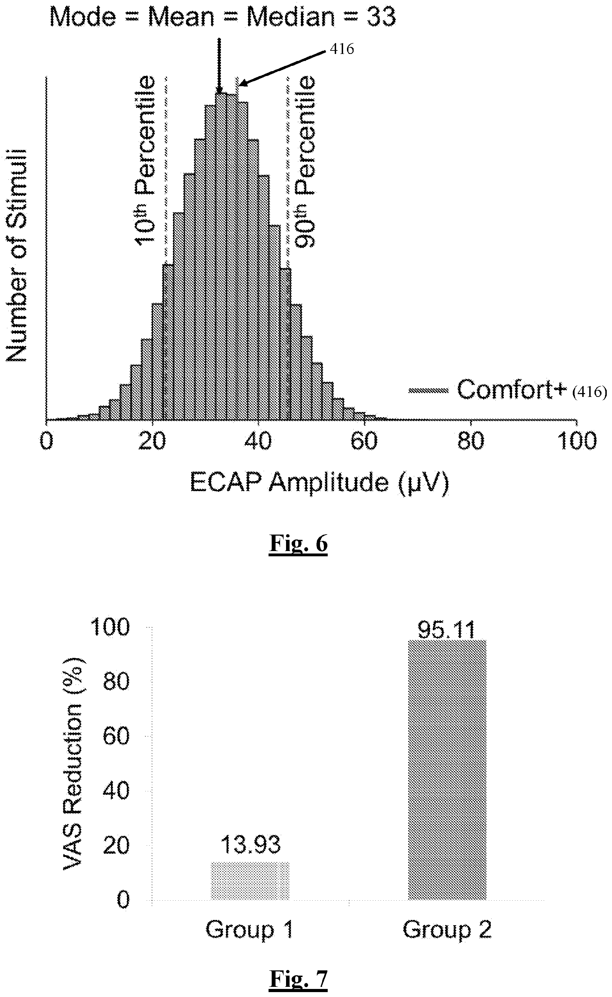

Of particular interest to the present invention, each individual histogram of ECAP amplitude in the Therapy Log 512 can be displayed and analysed as shown in Fig. 6, whether the device 100 is operating in open loop mode (no feedback) or closed loop mode (feedback control of stimulation based on observed ECAP amplitude). The present invention recognises that obtaining a sufficient number of individual neural activation measurements provides a sufficient sample size of such data to permit one or more statistical measures of the neural activation to be determined. For example, statistical measures of the ECAP amplitude histogram data which can be extracted include the minimum value, maximum value, mean, median, mode, and standard deviation. In more detail, particularly preferred statistical measures in accordance with some embodiments of the invention include (1) the mode, (2) the ratio of the mode to the comfort+ level (mode/comfort+), and the difference between the 90th percentile and 10th percentile, divided by the median ((90th-10th)/median).

Table I below describes how some such statistics are calculated on individual histograms in accordance with some embodiments of the invention. Note that the units (except for the Total Number of Stimulations) are the same units as used by the x-axis, i.e. $\mu$V for the FBV.

Table I: Histogram definitions and parameter information

| | |
|---|---|
| Bin Width | Specifies the range of the unit for each bin ($b$). The bin width is calculated from a *numerator* ($n$) and a *denominator in bit shifts* ($d$) field using the formula: $$b = \frac{2^d}{n}$$ |
| Number of Bins | The number of elements in the "Bins" array. |
| Bins | An array of stimulations for a given range. The range is calculated using the formula: [$ib$, ($i$ + 1)$b$) where i is the index of the element in the array and b is the bin width. |
| Total Number of Stimulations | $$C = \sum_{i=0}^{N-1} c_i$$ |
| Minimum (left-hand-edge of the smallest bin with a count greater than 0) | $ib$ for min $i$ where $c_i > 0$ |
| Maximum (right-hand-edge of the largest bin with a count greater than 0) | ($i$ + 1)b for max i where $c_i > 0$ |
| Mean ($\mu$) | $$\mu = \frac{1}{C} \sum_{i=0}^{N-1} c_i \left( \frac{ib + (i+1)b}{2} \right)$$ |
| Median ($M_d$) | $$\sum_{i=0}^{M_d/b} c_i = \frac{C+1}{2}$$ |
| Mode ($M_0$) | $$M_0 = \left( \frac{ib + (i+1)b}{2} \right)$$ for i where $c_i = \max_{i \in [0,N-1]} c_i$ |
| Standard Deviation ($\sigma$) | $$\sigma = \sqrt{\frac{1}{N} \sum_{i=0}^{N-1} c_i \left( \left( \frac{ib + (i+1)b}{2} \right) - \mu \right)^2}$$ |

Example 1: A closed-loop SCS system measured human spinal cord evoked compound action potentials (ECAPs) in real time, allowing correlation of dorsal column activation (ECAP amplitude) with patient-reported outcomes, thus providing objective measures of SCS effectiveness in pain management. Subjects were implanted with a new SCS system in a clinical trial study. Histograms of the distribution of ECAP amplitudes were extracted from each subject's device at the 12-month visit and are representative of everyday use. An example histogram is shown in **Fig. 6.** Statistical measures of these histograms were performed to form a neural response profile (NRP). The NRP is a quantitative summary of dorsal column response to electrical stimulus over a defined period (Table 2). Pain reduction using the Visual Analog Scale (VAS) and NRPs were summarized, for two groups of participants: Group 1, patients with minimally important pain relief, being those with VAS reduction <30% at the 12-month visit (N=5, Table 3(A)); and Group 2, patients with substantial pain relief, being those with VAS reduction ≥50% at the 12-month visit (N=18, Table 3(A)). **Fig. 7** illustrates the respective VAS reduction observed in these two groups.

Table 2: Neural Response Profile Variables

| Variable | Statistical Description |
|---|---|
| Mode | Most frequent ECAP amplitude |
| Mode/Comfort+ | Subject's in-clinic comfort level normalized to the mode: values close to 1 indicate a close match. Comfort+ may be replaced with any suitable clinician prescribed activation level |
| (90th-10th)/Median | The interdecile spread of the NRP normalized to the median indicating the NRP width and ECAP amplitude variability |
| Mean/Median | Measures the symmetry of the NRP: 1 indicates symmetry |

Initial observations indicate notable differences between cohorts in NRP values (Table 3; Figures 7-11). When considered subjectively, Fig. 7 shows that this division gives an average VAS reduction of 14% in Group 1, and a much improved VAS reduction in Group 2 of 95%.

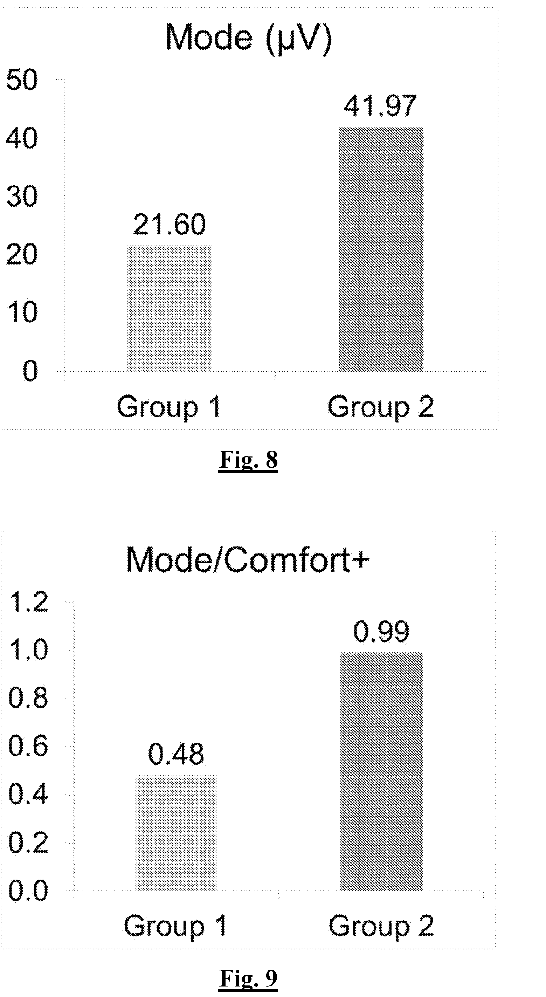

**Fig. 8** illustrates the average mode of ECAP amplitude for each SCS trial group. As shown, the average of the mode ECAP amplitude was twice as high in Group 2 as in Group 1. Thus, a relatively higher mode may be associated with greater efficacy of SCS.

**Fig. 9** illustrates the average of the mode/comfort+ ratio for each SCS trial group. As shown, the mode/comfort+ ratio was twice as high in Group 2 as in Group 1. Thus, a relatively higher mode/comfort+ ratio may be associated with greater efficacy of SCS.

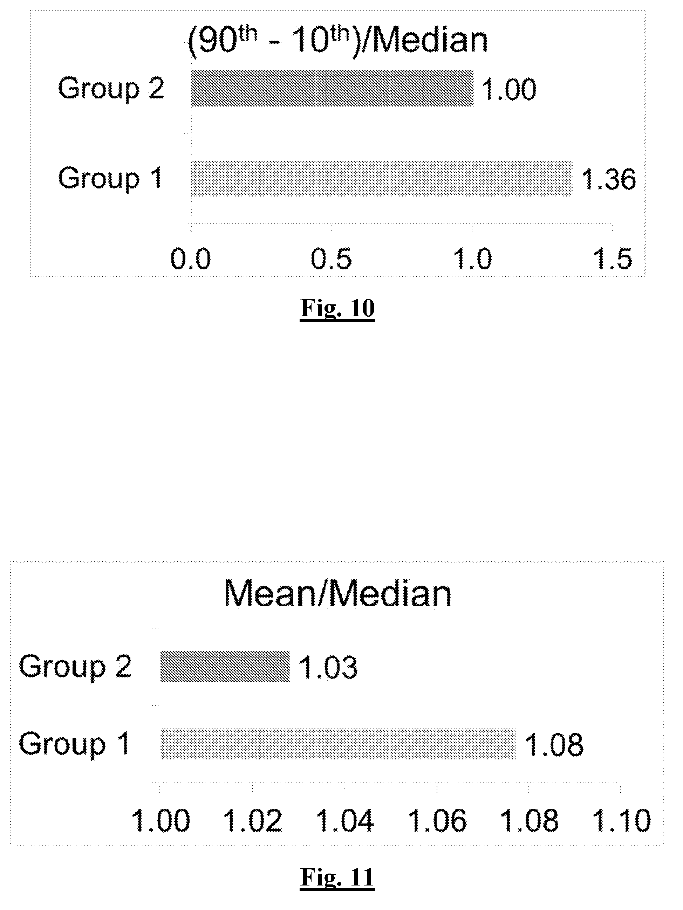

**Fig. 10** illustrates the average of the (90th-10th)/median ratio for each SCS trial group. As shown, in Fig. 10, the average of the (90th-10th)/median ratio was 1.4 for Group 1 and 1.0 for Group 2. Thus, a relatively narrower normalised interdecile range may be associated with increased SCS efficacy.

**Fig. 11** illustrates the average of the mean/median ratio for each SCS trial group. As shown, the mean/median ratio was 1.03 in Group 2 and 1.08 in Group 2. Thus, patients with substantial pain relief had more consistent spinal cord activation, so that greater asymmetry may be associated with reduced SCS efficacy. This may manifest as a mean/median ratio which deviates away from 1.0 in either direction, whether higher or lower.

Table 3: 12-Month VAS and NRP Variables Compared Between Group 1 (VAS reduction <30%) and Group 2 (VAS reduction ≥50%)

| | VAS Reduction (%) (A) | Mode (μV) (B) | Mode/Comfort+ (C) | (90th-10th)/Median (D) | Mean/Median (E) |
|---|---|---|---|---|---|
| VAS Reduction <30% (Group 1) | | | | | |
| N | 5 | 5 | 5 | 5 | 5 |
| Mean | 13.93 | 21.60 | 0.48 | 1.36 | 1.08 |
| Median | 15.46 | 16.50 | 0.38 | 1.31 | 1.04 |
| STD | 11.12 | 19.62 | 0.44 | 0.75 | 0.09 |
| VAS Reduction ≥50% (Group 2) | | | | | |
| N | 19 | 18 | 17 | 18 | 18 |
| Mean | 95.11 | 41.97 | 0,99 | 1.00 | 1.03 |
| Median | 97.47 | 27.75 | 0.70 | 0.99 | 1.01 |
| STD | 5.18 | 41.28 | 1.09 | 0.58 | 0.05 |

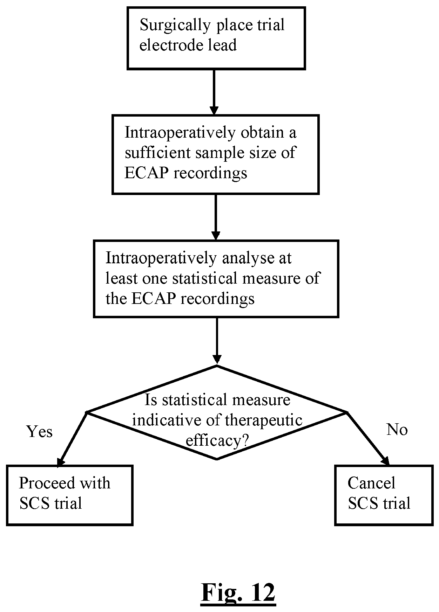

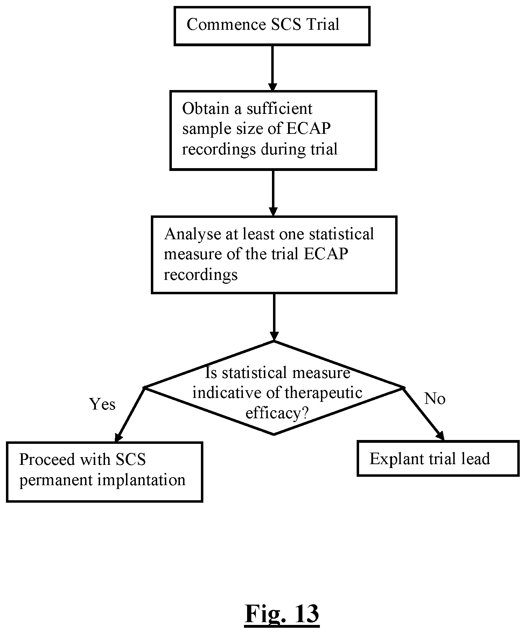

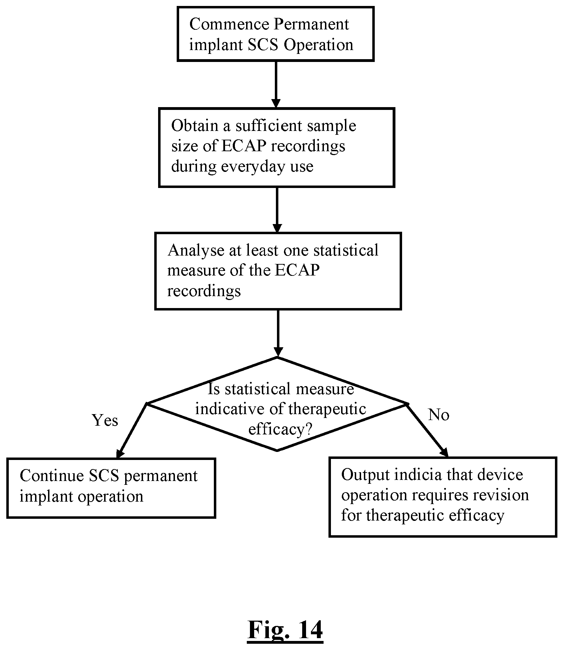

Example 1 thus provides human neurophysiological data suggesting that the magnitude of pain relief in SCS correlates with the magnitude of dorsal column activation, and is inversely related to the variability of activation. The NRP, comprising such statistical measures of neural activation, thus provides the first objective neurophysiological tool in SCS in contrast to past subjective measures. The NRP in turn may provide diagnostic support for predicting response to SCS, whether intraoperatively (Fig. 12), during SCS trials (Fig. 13) or during permanent implant stage (Fig. 14), and may thus in some embodiments assist in improving long-term success rates.

**Fig. 12** illustrates a procedure as may be executed in part by a software application in accordance with an embodiment of the invention. The application is configured to assess a statistically significant sample size of intraoperatively obtained ECAP recordings, in order to give a rapid and intraoperative output indication to clinicians as to whether the patient undergoing surgery is likely to benefit from an SCS trial. In cases where the patient's neural response profile is indicative that the patient will be a non-responder, the trial can be cancelled and the trial electrode lead can be removed, avoiding the costs and risks of a transcutaneous lead during a SCS trial.

**Fig. 13** illustrates a procedure as may be executed in part by a software application in accordance with an embodiment of the invention. The application is configured to assess a statistically significant sample size of ECAP recordings obtained during an SCS trial, in order to give an indication to clinicians as to whether the patient undergoing the trial is likely to benefit from permanent SCS implantation. In cases where the patient's neural response profile is indicative that the patient will be a non-responder, the trial can be concluded and the trial electrode lead can be removed, avoiding the costs and risks of a permanent SCS implant.

**Fig. 14** illustrates a procedure as may be executed in part by a software application in accordance with an embodiment of the invention. The application is configured to assess a statistically significant sample size of ECAP recordings obtained during normal day to day operation of a permanent SCS implant, in order to give an indication to clinicians or to the user as to whether the implant recipient is continuing to benefit from permanent SCS implantation. In cases where the patient's neural response profile is indicative that the patient has been a non-responder for that time period, the device operation can be revised or optimised, or the patient may be referred for consideration for explantation of the permanent SCS implant.

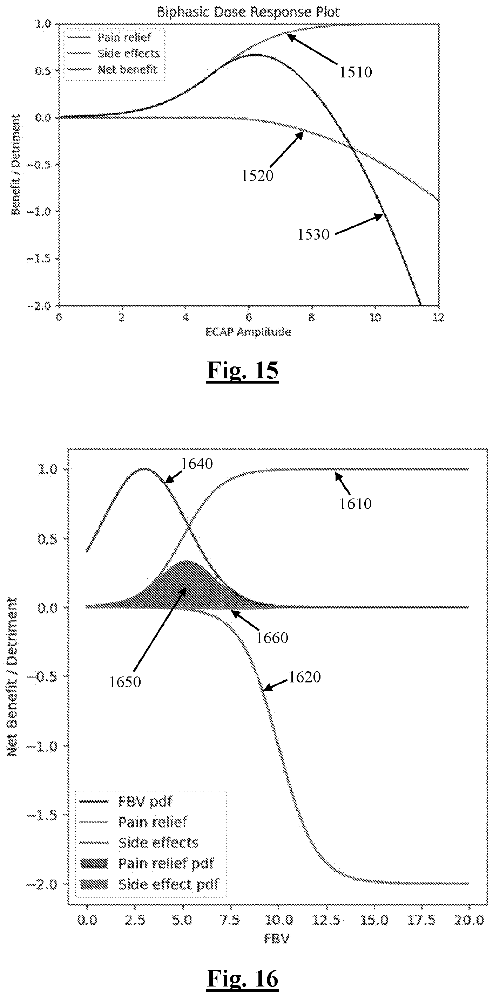

Without intending to be limited by theory, it is noted that one hypothesis for optimal therapeutic efficacy involves considering a dose response plot, as illustrated in **Fig. 15.** A dose response benefit curve 1510 reflects the increasing beneficial pain relief effected by increasing neural recruitment, while a dose-response detriment curve 1520 reflects the harm caused by excessive stimulation. Such harm may comprise worsening of the disease state and/or immediate pain. Each individual stimulus can produce a beneficial part and a detrimental part, which sum to provide a net benefit as indicated by curve 1530. The x-axis units are arbitrary for illustrative purposes, but can be tens of microvolts of ECAP in a typical situation and will vary from one patient to the next.

The onset of therapeutic benefit is measured as the patient's threshold. A stimulus that does not recruit any ECAP does not provide benefit. The onset of harm might coincide with the patient's comfort+ value or their maximum value or may fall at a value in between. This diagram matches clinical experience, where for low values of stimulation (say, ECAP Amplitude < 2 in Fig. 15) there is no benefit, but if the stimulation is too high (ECAP Amplitude > 9) then the patient would prefer no stimulation at all, so the detrimental side effects exceed the beneficial pain relief above this point and the net benefit falls below zero, i.e. there is a net detriment. The black line 1530 is referred to herein as a "biphasic dose response curve".

The present invention can be considered as recognising that when repeated stimuli are presented, they add to produce a net benefit and harm over time. These are found by multiplying the probability density function of the ECAP amplitude (i.e. the histogram) by the benefit and detriment curves, and summing the total area produced (where detriment takes negative values).

In **Fig. 16,** the green line 1610 represents the benefit dose/response curve and the red line 1620 represents the harm dose response curve. The blue line 1640 illustrates the ECAP probability density function (PDF), which in preferred embodiments is captured as a histogram by the implanted device. The green area 1650 represents net benefit as a probability density function and the red area 1660 represents net harm in a similar manner.

By varying the stimulation amplitude, the proportion of benefit and harm areas (1650 : 1660) varies. In Fig. 16 it can be seen that some benefit is derived and little harm. However, a significant portion of the recruited ECAPs fall below or near the onset of the benefit curve 1610 and are thus ineffectual and this portion of the therapy might thus be considered to be wasted.

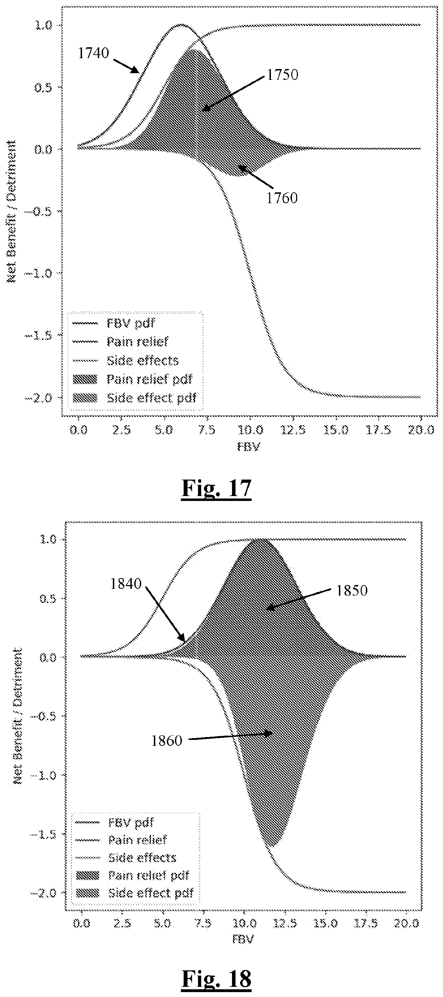

In **Fig. 17,** the stimulation level has been increased causing ECAP pdf 1740 to be positioned further to the right than 1640. This causes the benefit 1750 to increase to close to an optimum value, significantly larger than benefit 1650. However, the net detriment 1760 has also increased and is significantly larger than detriment 1660.

In **Fig. 18,** the stimulation has been further increased causing ECAP pdf 1840 to shift further to the right again. The benefit 1850 has reached a plateau and is not significantly greater than benefit 1750, whereas the harm 1860 has increased significantly beyond detriment 1760, to the point that detriment 1860 outweighs the benefit 1850.

Figs 16-18 thus illustrate that knowledge of the benefit and detriment dose-response curves permits the optimum stimulation amplitude to be determined.

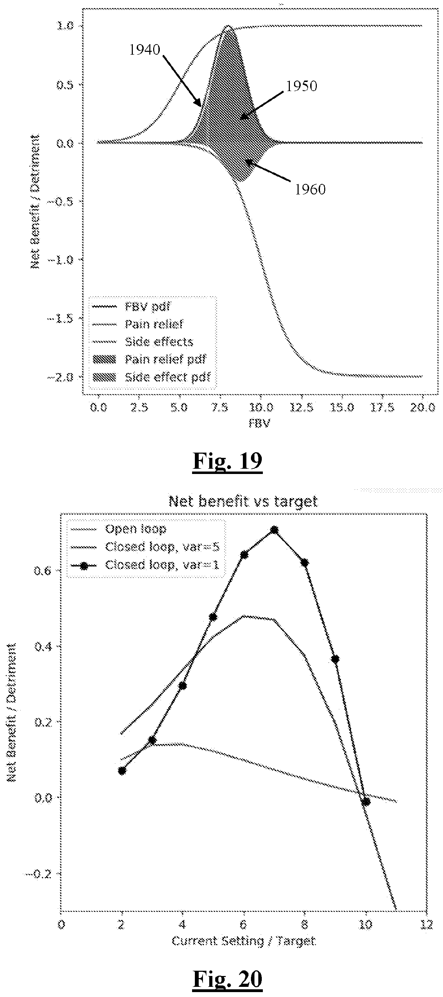

Embodiments of the present invention can further be considered to recognise that a narrower ECAP pdf 1640/1740/1840, i.e. a narrower histogram, is a desirable goal of stimulation as this improves the system's ability to increase the proportion of beneficial stimuli as compared to detrimental stimuli. This is illustrated in Figs 19 and 20.

In **Fig. 19,** ECAP pdf 1940 is centred around a value of about 8.5, greater than the centre value of ECAP pdfs 1640 and 1740. However, as the dispersion or variance of the histogram 1940 is reduced compared to ECAP pdfs 1640/1740/1840, fewer low amplitude ECAPs occur and thus fewer stimuli result in wasted or ineffectual therapy. Consequently, the benefit curve 1950 maximises the benefit of the applied stimuli and nearly conforms to curve 1940. Moreover, the narrow profile also results in fewer large amplitude ECAPs and thus a reduced amount of highly detrimental stimuli, and a small overall amount of detriment as reflected by curve 1960.

**Fig 20** compares open loop (green), closed loop with a variance of 5 and closed loop with a variance of 1. For each histogram, the net benefit and detriment were summed and added. This shows that, when the therapeutic efficacy of neurostimulation is evaluated by way of such a theoretical dose response curve, the narrower histogram produces the greatest benefit as it fits the greatest proportion of stimuli between the peak benefit curve and the onset of detriment. Open loop operation delivers the poorest net benefit.

It will be appreciated by persons skilled in the art that numerous variations and/or modifications may be made to the invention as defined by the claims herein. The present embodiments are, therefore, to be considered in all respects as illustrative and not limiting or restrictive.

**Claims**

1. A system for assessing therapeutic efficacy of electrical neurostimulation therapy, the system comprising:

   a neuromodulation device (100) configured to deliver the electrical neurostimulation therapy;
   measurement circuitry (128) configured to capture recordings of neural responses evoked by the electrical neurostimulation therapy; and
   a processor configured to process the recordings of the neural responses evoked by the delivered electrical neurostimulation therapy, in order to obtain a plurality of measures of neural activation in response to a plurality of electrical stimuli delivered over time, the processor further configured to calculate from the plurality of measures of neural activation in response to the plurality of delivered electrical stimuli at least one statistical measure of neural activation by the plurality of delivered electrical stimuli, the processor further configured to produce from the at least one statistical measure of neural activation by the plurality of delivered electrical stimuli an indication of therapeutic efficacy, the processor further configured to output the indication of therapeutic efficacy.

2. The system of claim 1 wherein the recordings of neural responses each comprise a recording of at least a portion of an evoked compound action potential (ECAP) evoked by a respective one of the plurality of delivered electrical stimuli, and wherein the measures of neural activation by the plurality of delivered electrical stimuli each comprise a measure of ECAP amplitude evoked by the respective one of the plurality of delivered electrical stimuli.

3. The system of claim 1 or claim 2 wherein the processor is configured to:

   assemble the measures of neural activation by the plurality of delivered electrical stimuli into a neural activation histogram, the neural activation histogram representing neural activation by the plurality of delivered electrical stimuli; and
   determine the at least one statistical measure of neural activation by the plurality of delivered electrical stimuli from the neural activation histogram.

4. The system of any one of claims 1 to 3 wherein the at least one statistical measure of neural activation by the plurality of delivered electrical stimuli calculated from the plurality of measures comprises a mode of the plurality of measures.

5. The system of any one of claims 1 to 4 wherein the at least one statistical measure of neural activation by the plurality of delivered electrical stimuli calculated from the plurality of measures comprises a mode/comfort+ ratio of the plurality of measures, where comfort+ is a clinician prescribed level of desirable neural activation.

6. The system of any one of claims 1 to 5 wherein the at least one statistical measure of neural activation by the plurality of delivered electrical stimuli calculated from the plurality of measures comprises a normalised interdecile range of the plurality of measures.

7. The system of claim 6 wherein the interdecile range is normalised by dividing each of the plurality of measures by at least one of a median, mean or mode of the plurality of measures.

8. The system of any one of claims 1 to 7 wherein the at least one statistical measure of neural activation by the plurality of delivered electrical stimuli calculated from the plurality of measures comprises a measure of distribution asymmetry.

9. The system of claim 8 wherein the measure of distribution asymmetry comprises at least one of: a mean/median ratio of the plurality of measures, a measure of dispersion of the plurality of measures, a coefficient of variation of the plurality of measures, a kurtosis of the plurality of measures, and a skew of the plurality of measures.

10. The system of any one of claims 1 to 9 wherein the plurality of measures are measures of evoked compound action potential (ECAP) amplitude evoked by a respective one of the plurality of delivered electrical stimuli at a comfort+ level, in a pre-determined posture, and wherein the processor is configured to calculate the at least one statistical measure of neural activation by the plurality of delivered electrical stimuli as being a coefficient of variation of the plurality of measures.

11. The system of claim 10 wherein the processor is configured to produce a greater indication of therapeutic efficacy when the coefficient of variation is small, and to produce a lesser indication of therapeutic efficacy when the coefficient of variation is large.

12. The system of any one of claims 1 to 11 wherein the processor is configured to derive two or more statistical measures of neural activation by the plurality of delivered electrical stimuli from the plurality of measures of neural activation, and is further configured to produce a combinatorial indication of therapeutic efficacy from the two or more statistical measures of neural activation by the plurality of delivered electrical stimuli.

13. The system of any one of claims 1 to 12, further configured to use the indication of therapeutic efficacy as a binary indicator to indicate whether permanent implantation should proceed.

14. The system of any one of claims 1 to 12 wherein the processor is configured to perform an automated procedure for improving therapeutic efficacy, by iteratively revising the therapy to seek an improvement in the indication of therapeutic efficacy, wherein the processor is configured to iteratively revise the therapy by: (i) measuring a first indication of therapeutic efficacy with a feedback loop gain set at a first value, (ii) adjusting feedback loop gain from the first value to a second value, (iii) measuring a second indication of therapeutic efficacy and (iv) if the second indication indicates higher therapeutic efficacy than the first indication, retaining the second value of loop gain for ongoing use.

15. A non-transitory computer readable medium for assessing therapeutic efficacy of electrical neurostimulation therapy, comprising instructions which, when executed by one or more processors, causes performance of the following:

   delivering, by a stimulation electrode of a stimulator device, electrical neurostimulation therapy;
   capturing, by a sense electrode of the stimulator device, recordings of neural responses evoked by the electrical neurostimulation therapy;
   processing, by the one or more processors, the recordings of the neural responses evoked by the delivered electrical neurostimulation therapy, in order to obtain a plurality of measures of neural activation in response to a plurality of electrical stimuli delivered over time,
   calculating, by the one or more processors, from the plurality of measures of neural activation in response to the plurality of delivered electrical stimuli at least one statistical measure of neural activation by the plurality of delivered electrical stimuli,
   producing, by the one or more processors, from the at least one statistical measure of neural activation by the plurality of delivered electrical stimuli an indication of therapeutic efficacy; and
   outputting, by the one or more processors, the indication of therapeutic efficacy.

**Patentansprüche**

1. System zur Bestimmung der therapeutischen Wirksamkeit von elektrischer Neurostimulationstherapie, wobei das System umfasst:

   eine Neuromodulationsvorrichtung, die zur Abgabe der elektrischen Neurostimulationstherapie konfiguriert ist;

eine Messschaltung, die so konfiguriert ist, dass sie Aufzeichnungen von neuronalen Reaktionen erfasst, die durch die elektrische Neurostimulationstherapie hervorgerufen werden; und

einen Prozessor, der so konfiguriert ist, dass er die Aufzeichnungen der durch die abgegebene elektrische Neurostimulationstherapie hervorgerufenen neuralen Reaktionen verarbeitet, um eine Vielzahl von Messwerten der neuralen Aktivierung als Reaktion auf eine Vielzahl von im Laufe der Zeit abgegebenen elektrischen Stimuli zu erhalten, wobei der Prozessor ferner so konfiguriert ist, dass er aus der Vielzahl von Messwerten der neuralen Aktivierung als Reaktion auf die Vielzahl von abgegebenen elektrischen Stimuli mindestens ein statistisches Maß der neuralen Aktivierung durch die Vielzahl von abgegebenen elektrischen Stimuli berechnet, wobei der Prozessor ferner so konfiguriert ist, dass er aus dem mindestens einen statistischen Maß der neuralen Aktivierung durch die Vielzahl der abgegebenen elektrischen Stimuli eine Angabe der therapeutischen Wirksamkeit erzeugt, wobei der Prozessor ferner so konfiguriert ist, dass er die Angabe der therapeutischen Wirksamkeit ausgibt.

2. System nach Anspruch 1, wobei die Aufzeichnungen der neuralen Reaktionen jeweils eine Aufzeichnung von mindestens einem Teil eines evozierten zusammengesetzten Aktionspotentials (ECAP) umfassen, das durch einen jeweiligen aus der Vielzahl der abgegebenen elektrischen Stimuli evoziert wird, und wobei die Messwerte der neuralen Aktivierung durch die Vielzahl der abgegebenen elektrischen Stimuli jeweils einen Messwert der ECAP-Amplitude umfassen, die durch den jeweiligen aus der Vielzahl der abgegebenen elektrischen Stimuli evoziert wird.

3. Das System nach Anspruch 1 oder Anspruch 2, wobei der Prozessor konfiguriert ist, um:

die Messwerte der neuralen Aktivierung durch die Vielzahl der abgegebenen elektrischen Stimuli zu einem Histogramm der neuralen Aktivierung zusammenzusetzen, wobei das neurale Aktivierungshistogramm die neurale Aktivierung durch die Vielzahl der abgegebenen elektrischen Stimuli darstellt; und

das mindestens eine statistische Maß der neuralen Aktivierung durch die Vielzahl der abgegebenen elektrischen Stimuli aus dem Histogramm der neuralen Aktivierung zu bestimmen.

4. System nach einem der Ansprüche 1 bis 3, wobei das mindestens eine statistische Maß der neuralen Aktivierung durch die Vielzahl der abgegebenen elektrischen Stimuli, das aus der Vielzahl der Messwerte berechnet wird, einen Modalwert der Vielzahl der Messwerte umfasst.

5. System nach einem der Ansprüche 1 bis 4, wobei das mindestens eine statistische Maß der neuralen Aktivierung durch die Vielzahl abgegebener elektrischer Stimuli, das aus der Vielzahl von Messwerten berechnet wird, ein Modalwert/Komfort+-Verhältnis der Vielzahl von Messwerten umfasst, wobei Komfort+ ein vom Kliniker vorgeschriebenes Niveau der erwünschten neuralen Aktivierung ist.

6. System nach einem der Ansprüche 1 bis 5, wobei das mindestens eine statistische Maß der neuralen Aktivierung durch die Vielzahl abgegebener elektrischer Stimuli, berechnet aus der Vielzahl der Messwerte, einen normalisierten Interdezilbereich der Vielzahl der Messwerte umfasst.

7. System nach Anspruch 6, wobei der Interdezilbereich normalisiert wird, indem jeder der Vielzahl der Messwerte durch mindestens einen von Median, Mittelwert oder Modalwert der Vielzahl der Messwerte geteilt wird.

8. System nach einem der Ansprüche 1 bis 7, wobei das mindestens eine statistische Maß der neuronalen Aktivierung durch die Vielzahl der abgegebenen elektrischen Stimuli, das aus der Vielzahl der Messwerte berechnet wird, ein Maß der Verteilungsasymmetrie umfasst.

9. System nach Anspruch 8, wobei das Maß der Verteilungsasymmetrie mindestens eines der folgenden Merkmale umfasst: ein Mittelwert/Median-Verhältnis der Vielzahl von Messwerten, ein Maß der Streuung der Vielzahl von Messwerten, einen Variationskoeffizienten der Vielzahl von Messwerten, eine Kurtosis der Vielzahl von Messwerten und eine Schiefe der Vielzahl von Messwerten.

10. System nach einem der Ansprüche 1 bis 9, wobei die Vielzahl der Messwerte Messwerte der Amplitude des evozierten zusammengesetzten Aktionspotentials (ECAP) sind, das durch einen jeweiligen der Vielzahl der abgegebenen elektrischen Stimuli bei einem Komfort+-Level in einer vorbestimmten Haltung evoziert wird, und wobei der Prozessor so konfiguriert ist, dass er das mindestens eine statistische Maß der neuralen Aktivierung durch die Vielzahl der abgegebenen elektrischen Stimuli als Variationskoeffizient der Vielzahl der Messwerte berechnet.

11. System nach Anspruch 10, wobei der Prozessor so konfiguriert ist, dass er eine größere Angabe der therapeutischen Wirksamkeit erzeugt, wenn der Variationskoeffizient klein ist, und dass er eine geringere Angabe der therapeutischen Wirksamkeit erzeugt, wenn der Variationskoeffizient groß ist.

12. System nach einem der Ansprüche 1 bis 11, wobei der Prozessor so konfiguriert ist, dass er zwei oder mehr statistische Maße der neuralen Aktivierung durch die Vielzahl der abgegebenen elektrischen Stimuli aus der Vielzahl der Messwerte der neuralen Aktivierung ableitet, und ferner so konfiguriert ist, dass er eine kombinatorische Angabe der therapeutischen Wirksamkeit aus den zwei oder mehr statistischen Maßen der neuralen Aktivierung durch die Vielzahl der abgegebenen elektrischen Stimuli erzeugt.

13. Das System nach einem der Ansprüche 1 bis 12, das ferner so konfiguriert ist, dass es die Angabe der therapeutischen Wirksamkeit als binären Indikator verwendet, um anzuzeigen, ob die permanente Implantation fortgesetzt werden sollte.

14. System nach einem der Ansprüche 1 bis 12, wobei der Prozessor so konfiguriert ist, dass er ein automatisiertes Verfahren zur Verbesserung der therapeutischen Wirksamkeit durchführt, indem er die Therapie iterativ überarbeitet, um eine Verbesserung der Angabe der therapeutischen Wirksamkeit zu erreichen, wobei der Prozessor so konfiguriert ist, dass er die Therapie iterativ überarbeitet, durch: (i) Messen einer ersten Angabe der therapeutischen Wirksamkeit mit einer auf einen ersten Wert eingestellten Rückkopplungsschleifenverstärkung ist, (ii) Einstellen der Rückkopplungsschleifenverstärkung von dem ersten Wert auf einen zweiten Wert, (iii) Messen einer zweiten Angabe der therapeutischen Wirksamkeit und (iv) wenn die zweite Angabe eine höhere therapeutische Wirksamkeit als die erste Angabe anzeigt, Beibehalten des zweiten Wertes der Schleifenverstärkung für die weitere Verwendung.

15. Nicht-transitorisches computerlesbares Medium zur Bestimmung der therapeutischen Wirksamkeit einer elektrischen Neurostimulationstherapie, das Anweisungen enthält, die bei Ausführung durch einen oder mehrere Prozessoren Folgendes bewirken:

Abgeben einer elektrischen Neurostimulationstherapie durch eine Stimulationselektrode einer Stimulatorvorrichtung;
Erfassen von Aufzeichnungen neuronaler Reaktionen, die durch die elektrische Neurostimulationstherapie hervorgerufen werden, durch eine Sensorelektrode der Stimulatorvorrichtung;
Verarbeiten der Aufzeichnungen der neuralen Reaktionen, die durch die verabreichte elektrische Neurostimulationstherapie hervorgerufen werden, durch den einen oder die mehreren Prozessoren, um eine Vielzahl von Messwerten der neuralen Aktivierung als Reaktion auf eine Vielzahl von im Laufe der Zeit abgegebenen elektrischen Stimuli zu erhalten,
Berechnen von mindestens einem statistischen Maß der neuralen Aktivierung durch die Vielzahl der abgegebenen elektrischen Stimuli aus der Vielzahl der Messwerte der neuralen Aktivierung in Reaktion auf die Vielzahl der abgegebenen elektrischen Stimuli durch den einen oder die mehreren Prozessoren,
Erzeugen einer Angabe zur therapeutischen Wirksamkeit durch den einen oder die mehreren Prozessoren aus dem mindestens einen statistischen Maß der neuronalen Aktivierung durch die Vielzahl der abgegebenen elektrischen Stimuli; und
Ausgeben der Angabe der therapeutischen Wirksamkeit durch den einen oder die mehreren Prozessoren.

**Revendications**

1. Système d'évaluation de l'efficacité thérapeutique d'une thérapie de neurostimulation électrique, le système comprenant :

un appareil de neuromodulation (100) configuré pour délivrer la thérapie de neurostimulation électrique ;
un circuit de mesure (128) configuré pour saisir des enregistrements de réponses neuronales évoquées par la thérapie de neurostimulation électrique ; et
un processeur configuré pour traiter les enregistrements des réponses neuronales évoquées par la thérapie de neurostimulation électrique afin d'obtenir une pluralité de mesures d'activation neuronale en réponse à une pluralité de stimuli électriques délivrés au fil du temps, le processeur étant en outre configuré pour calculer à partir de la pluralité de mesures d'activation neuronale en réponse à une pluralité de stimuli électriques délivrés au moins une mesure statistique d'activation neuronale par la pluralité de stimuli électriques délivrés, le processeur étant en outre configuré pour produire à partir de l'au moins une mesure statistique d'activation neuronale par la pluralité de stimuli électriques délivrés une indication d'efficacité thérapeutique, le processeur étant en outre configuré pour sortir l'indication d'efficacité thérapeutique.

2. Système selon la revendication 1, dans lequel les enregistrements de réponses neuronales comprennent chacun un enregistrement d'au moins une partie d'un potentiel d'action composé évoqué (ECAP) évoqué par un respectif parmi la pluralité de stimuli électriques délivrés, et dans lequel les mesures d'activation neuronale par la pluralité de stimuli électriques délivrés comprennent chacune une mesure de l'amplitude d'ECAP évoquée par celui respectif parmi la pluralité de stimuli électriques délivrés.

3. Système selon la revendication 1 ou la revendication 2, dans lequel le processeur est configuré pour :

assembler les mesures d'activation neuronale par la pluralité de stimuli électriques délivrés dans un histogramme d'activation neuronale, l'histogramme d'activation neuronale représentant une activation neuronale par la pluralité de stimuli électriques délivrés ; et
déterminer l'au moins une mesure statistique d'activation neuronale par la pluralité de stimuli électriques délivrés à partir de l'histogramme d'activation neuronale.

4. Système selon l'une quelconque des revendications 1 à 3, dans lequel l'au moins une mesure statistique d'activation neuronale par la pluralité de stimuli électriques délivrés calculée à partir de la pluralité de mesures comprend un mode de la pluralité de mesures.

5. Système selon l'une quelconque des revendications 1 à 4, dans lequel l'au moins une mesure statistique d'activation neuronale par la pluralité de stimuli électriques délivrés calculée à partir de la pluralité de mesures comprend un rapport mode/confort+ de la pluralité de mesures, où confort+ est un niveau clinique prescrit d'activation neuronale désirable.

6. Système selon l'une quelconque des revendications 1 à 5, dans lequel l'au moins une mesure statistique d'activation neuronale par la pluralité de stimuli électriques délivrés calculée à partir de la pluralité de mesures comprend une gamme interdéciles normalisée de la pluralité de mesures.

7. Système selon la revendication 6, dans lequel la gamme interdécile est normalisée en divisant chacune de la pluralité de mesures par au moins un parmi une médiane, une moyenne ou un mode de la pluralité de mesures.

8. Système selon l'une quelconque des revendications 1 à 7, dans lequel l'au moins une mesure statistique de l'activation neuronale par la pluralité de stimuli électriques délivrés calculée à partir de la pluralité de mesures comprend une mesure d'asymétrie de distribution.

9. Système selon la revendication 8, dans lequel la mesure d'asymétrie de distribution comprend au moins un parmi : un rapport moyenne/médiane de la pluralité de mesures, une mesure de dispersion de la pluralité de mesures, un coefficient de variation de la pluralité de mesures, un kurtosis de la pluralité de mesures, et une asymétrie de la pluralité de mesures.

10. Système selon l'une quelconque des revendications 1 à 9, dans lequel la pluralité de mesures est constituée de mesures d'amplitude de potentiel d'action composé évoqué (ECAP) évoqué par un respectif de la pluralité de stimuli électriques délivrés au niveau confort+ dans une posture prédéterminée, et dans lequel le processeur est configuré pour calculer l'au moins une mesure statistique d'activation neuronale par la pluralité de stimuli électriques délivrés en tant que coefficient de variation de la pluralité de mesures.

11. Système selon la revendication 10, dans lequel le processeur est configuré pour produire une plus grande indication d'efficacité thérapeutique quand le coefficient de variation est petit, et pour produire une plus petite indication d'efficacité thérapeutique quand le coefficient de variation est grand.

12. Système selon l'une quelconque des revendications 1 à 11, dans lequel le processeur est configuré pour dériver au moins deux mesures statistiques d'activation neuronale par la pluralité de stimuli électriques délivrés à partir de la pluralité de mesures d'activation neuronale, et est configuré en outre pour produire une indication combinatoire d'efficacité thérapeutique à partir des au moins deux mesures statistiques d'activation neuronale par la pluralité de stimuli électriques délivrés.

13. Système selon l'une quelconque des revendications 1 à 12, configuré en outre pour utiliser l'indication d'efficacité thérapeutique comme indicateur binaire pour indiquer s'il faut effectuer une implantation permanente.

14. Système selon l'une quelconque des revendications 1 à 12, dans lequel le processeur est configuré pour effectuer une procédure automatisée pour améliorer une efficacité thérapeutique en révisant itérativement la thérapie pour chercher une amélioration de l'indication d'efficacité thérapeutique, le processeur étant configuré pour réviser itérativement la thérapie : (i) en mesurant une première indication d'efficacité thérapeutique avec un gain de boucle de rétroaction fixé à une première valeur, (ii) en ajustant le gain de boucle de rétroaction de la première valeur à une deuxième valeur, (iii) en mesurant une deuxième indication d'efficacité thérapeutique et (iv) si la deuxième indication indique une efficacité thérapeutique supérieure à la première indication, en conservant la deuxième valeur de gain de boucle de rétroaction pour un usage continu.

15. Support non transitoire lisible par ordinateur pour évaluer l'efficacité thérapeutique d'une thérapie de neurostimulation électrique comprenant des instructions qui, quand elles sont exécutées par au moins un processeur, provoquent la réalisation des étapes suivantes :

délivrance, par une électrode de stimulation d'un dispositif stimulateur, d'une thérapie de neurostimulation électrique ;

saisie, par une électrode de détection du dispositif stimulateur, d'enregistrements de réponses neuronales évoquées par la thérapie de neurostimulation électrique ;

traitement, par l'au moins un processeur, des enregistrements des réponses neuronales évoquées par la thérapie de neurostimulation électrique délivrée afin d'obtenir une pluralité de mesures d'activation neuronale en réponse à une pluralité de stimuli électriques délivrés au fil du temps ;

calcul, par l'au moins un processeur, à partir de la pluralité de mesures d'activation neuronale en réponse à la pluralité de stimuli électriques délivrés, d'au moins une mesure statistique d'activation neuronale par la pluralité de stimuli électriques délivrés ;

production, par l'au moins un processeur, à partir de l'au moins une mesure statistique d'activation neuronale par la pluralité de stimuli électriques délivrés, d'une indication d'efficacité thérapeutique ; et

sortie, par l'au moins un processeur, de l'indication d'efficacité thérapeutique.

**Fig. 1**

**Fig. 2**

**Fig. 3**

**Fig. 4A**

**Fig. 4B**

**Fig. 5**

Fig. 6

Fig. 7

**Fig. 8**

**Fig. 9**

**(90th – 10th)/Median**

Group 2 ▨ 1.00

Group 1 ▨ 1.36

0.0    0.5    1.0    1.5

**Fig. 10**

**Mean/Median**

Group 2 ▨ 1.03

Group 1 ▨ 1.08

1.00   1.02   1.04   1.06   1.08   1.10

**Fig. 11**

Surgically place trial
electrode lead

Intraoperatively obtain a
sufficient sample size of
ECAP recordings

Intraoperatively analyse at
least one statistical measure of
the ECAP recordings

Is statistical measure
indicative of therapeutic
efficacy?

Yes

No

Proceed with
SCS trial

Cancel
SCS trial

**Fig. 12**

```
┌─────────────────────────┐
│   Commence SCS Trial    │
└─────────────────────────┘
             │
             ▼
┌─────────────────────────┐
│  Obtain a sufficient    │
│  sample size of ECAP    │
│  recordings during trial│
└─────────────────────────┘
             │
             ▼
┌─────────────────────────┐
│ Analyse at least one    │
│ statistical measure of  │
│ the trial ECAP          │
│ recordings              │
└─────────────────────────┘
             │
             ▼
        ╱─────────╲
       ╱ Is statistical measure ╲
Yes   ╱  indicative of therapeutic  ╲   No
  ◄──╱        efficacy?              ╲──►
      ╲                             ╱
       ╲───────────────────────────╱
   │                                  │
   ▼                                  ▼
┌──────────────────────┐   ┌─────────────────────┐
│ Proceed with SCS     │   │  Explant trial lead │
│ permanent implantation│   └─────────────────────┘
└──────────────────────┘
```

**Fig. 13**

```
┌─────────────────────────┐
│ Commence Permanent      │
│ implant SCS Operation   │
└─────────────────────────┘
            │
            ▼
┌─────────────────────────┐
│ Obtain a sufficient     │
│ sample size of ECAP     │
│ recordings during       │
│ everyday use            │
└─────────────────────────┘
            │
            ▼
┌─────────────────────────┐
│ Analyse at least one    │
│ statistical measure of  │
│ the ECAP recordings     │
└─────────────────────────┘
            │
            ▼
      Is statistical measure
      indicative of therapeutic
            efficacy?
   Yes                      No
```

Continue SCS permanent implant operation

Output indicia that device operation requires revision for therapeutic efficacy

**Fig. 14**

**Fig. 15**

**Fig. 16**

**Fig. 17**

**Fig. 18**

**Fig. 19**

**Fig. 20**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016077882 A
- WO 2017173493 A
- WO 2012155183 A
- WO 2017219096 A1